# EUROPEAN PATENT APPLICATION

(11) **EP 2 452 707 A1**
(43) Date of publication of application: **16.05.2012**
(21) Application number: 10796759.8
(22) Date of filing: 08.07.2010
(51) Int. Cl.: A61M 5/00, A61B 5/05

(54) **SU-8 MICRONEEDLES FOR MONITORING AND STIMULATING NEURONS**

(30) Priority: 09.07.2009 ES 200930430
(71) Applicant: Ikerlan, S. Coop., 20500 Mondragon (ES); Consejo Superior De Investigaciones Científicas, 28006 Madrid (ES)
(72) Inventor: GABRIEL, Gemma, E-08027 Barcelona (ES); GUIMERA, Anton, E-08720 Villafranca Del Penedes (Barcelona) (ES); MENENDEZ DE LA PRIDA,, Liset, E-28012 Madrid (ES); FERNANDEZ LEDESMA, Luis José, E-01010 Vitoria (Alava) (ES); TIJERO SERNA, María, E-20550 Aretxabaleta (Guipuzcua) (ES); ALTUNA LETAMENDI, Ane, E-20500 Arrasate (Guipuzcoa) (ES); VILLA, Rosa, E-08015 Barcelona (ES); BERGANZO RUIZ, Javier, E-01010 Vitoria (Alava) (ES)
(74) Representative: Carvajal y Urquijo, Isabel
(86) International application number: PCT/ES2010/070472
(87) International publication number: WO 2011/004047

(57) **Abstract**

The invention relates to an SU-8 microneedle for monitoring and stimulating neurons, having a thickness less than 100 micrometers and a length of 50 micrometers to 10 centimeters, the manufacturing method of which allows removing the microneedle from the substrate without using mechanical means due to the initial coating of a rigid substrate with an aluminium layer as a sacrificial layer and the final chemical etching of the aluminium layer for chemically removing the microneedle obtained in known intermediate photolithographic manufacturing steps.

## Description

### Field of the Invention

The present invention relates to designing and manufacturing SU-8 microneedles having a thickness less than 100 micrometers and a length which can range from 50 micrometers to 10 centimeters, for use in monitoring and/or stimulating neurons in brain-machine interface devices.

### Background of the Invention

Different methods are used today for obtaining microneedles for use in biomedicine, the materials making them up and their manufacturing method, which determines the hardness and fragility thereof and their use in one application or another, being of great importance.

In the case of monitoring organs such as liver or kidneys, needles with sufficient rigidity are required, so as to be able to be introduced through the hard outer layers. Therefore until now, work in obtaining needles having thicknesses of the order of several tenths of a millimeter has been done using both rigid materials such as silicon, and more flexible materials such as different polymers. Unfortunately, the neurological application of these devices is limited. Manipulating and stimulating neurons by means of microneedles is a very delicate and risky procedure not requiring high rigidity given the low mechanical strength of neural tissue. However, this application requires very thin materials to minimize damage during insertion; they must be flexible to prevent breakages during use and at the same time be sufficiently rigid to assure an appropriate insertion into the neural tissue. Until now no microneedle capable of meeting all these requirements is known.

Today, the technology for manufacturing microneedles for neurological application almost exclusively uses silicon as the substrate. This material allows different manufacturing processes assuring the production of thin devices. This silicon ability has traditionally prevented the use of other more biocompatible and flexible materials. Thus, no manufacturing process is known today which allows using polymers for designing thin microneedles, assuring the monopoly of silicon in the field. Nevertheless, silicon microneedles are shown to be hardly suitable for use in chronic implants associated with brain-machine interface devices and they are extraordinarily fragile and have a large risk of breaking inside the brain itself once implanted.

The polymer needles manufactured until now for use in organs such as liver or kidneys are obtained by the photolithography of a negative photoresin deposited on a rigid substrate. However, this method is not viable if one attempts to manufacture microneedles having thicknesses less than 100 micrometers, because it entails mechanically removing the already manufactured microneedle with the resulting risk of breaking. In this application a new manufacturing method for manufacturing microneedles having thicknesses less than 100 micrometers is developed using the SU-8 polymer, a photoresin widely used in microelectromechanical system applications, as the substrate

### Description of the Invention

The present invention relates to microneedles having a thickness less than 100 micrometers, manufactured using SU-8 as the structural material, for use in monitoring and/or stimulating neural tissue. The manufacturing method allows removing the microneedle from the substrate through chemical means thus preventing the use of mechanical methods and reducing the risk of breaking. This method uses a sacrificial aluminium layer for the initial coating of a rigid substrate, on which SU-8 is deposited as a negative resin. After a series of intermediate steps already known in the lithographic micro-electrode patterns manufacturing, the chemical etching of the sacrificial layer for removing the microneedle having the desired thickness is performed. This further allows manufacturing needles having a length which can be as long as 10 centimeters.

The SU-8 needles thus manufactured have many advantages over the only technology currently available which uses silicon as the substrate. Not only do they have a greater mechanical behavior and greater biocompatibility, solving part of the problems associated with the silicon without being detrimental to their performances, but they implicitly entail the possibility of introducing further improved technologies in the interface with the nerve tissue. Due to the method described, the electrodes adhered to the SU-8 microneedles are internalized within the polymer a few micrometers in depth below the tip surface, thus facilitating the subsequent deposition of materials which optimize the recording and electrical stimulation. Recent studies have proven the advantages of depositing carbon nanotubes on the micro-electrodes for improving not only their electrical capacity, but also the interaction with the neural tissue (Keefer EW, Botterman BR, Romero MI, Rossi AF, Gross GW. Carbon nanotube coating improves neuronal recordings, Nature Nanotechnol. 2008 Jul;3(7):434-9). While in the case of the silicon microneedles, designing additional methods would be necessary to facilitate coating the electrodes, the present manufacturing method for manufacturing SU-8 microneedles produces devices ready to be treated with current deposition methods.

### Brief Description of the Drawings

A set of drawings aiding to better understand the invention which is specifically related with an embodiment of said invention which is depicted as a non-limiting example thereof is described briefly below.

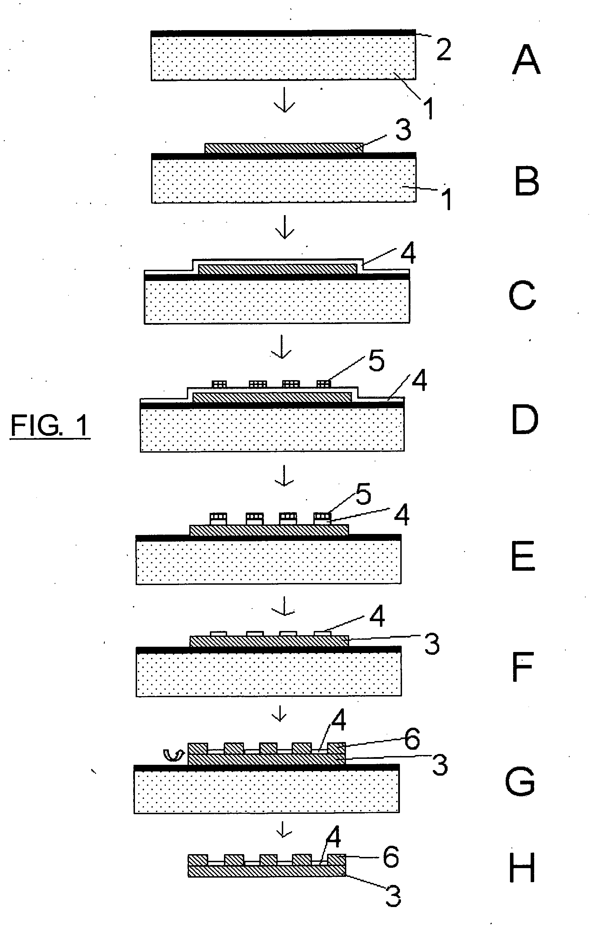

Figure 1 shows a diagram of the manufacturing method for manufacturing the microneedles of the invention. A series of numerical references corresponding with the following elements are represented in said figure:
   1.- Rigid substrate
   2.- Aluminium layer
   3.- Negative SU-8 photoresin
   4.- Cr/Au layer
   5.- Positive photoresin
   6.- Passivation layer

### Description of a Preferred Embodiment of the Invention

The present invention relates to SU8 microneedles having a thickness less than 100 micrometers and a length of 50 micrometers to 10 centimeters, for use in biomedicine neurological procedures such as neuron monitoring and stimulation.

The manufacturing method for manufacturing SU-8 microneedles is critical, since the thinness of these devices complicates their production without producing breakages with the current manufacturing methods. To that end the microneedles of the invention are manufactured by means of known photolithographic methods, with the difference that in a first step,(A), depicted in Figure 1, an aluminium layer (2) is deposited on a rigid substrate (1), in a second step (B) a negative SU8 photoresin (3) is placed on the aluminium layer (2). From this step the method continues in several known steps (C-F) for obtaining microneedles in which the SU-8 and aluminium surface are treated with a Cr/Au layer (4) which allows configuring different micro-electrode spatial patterns. A next layer of 1.8 µm-thick positive S1818 photoresin (5) which allows protecting the Cr/Au layer (4) is subsequently deposited resulting in electrodes in the desired areas. The S1818 photoresin (5) is subsequently removed and a passivation layer (6) is added in step G. Finally the microneedles are removed by chemical etching which allows preventing the breakages produced when using mechanical removing means currently used for thicker needles.

By means of the mentioned method, very thin microneedles can be obtained without breaking, the characteristics of which allow them to be used in neurological procedures such as monitoring and stimulating small neuron populations in brain-machine interface devices.

## Claims

1. A microneedle for monitoring and stimulating neurons, **characterized by** using a negative SU8 microresin as a substrate and by having thicknesses less than 100 micrometers.

2. A manufacturing method for manufacturing microneedles described in claim 1, **characterized by** comprising the steps of:
• coating a rigid substrate (1) with an aluminium layer (2)
• depositing a negative SU8 photoresin layer (3)
• obtaining small, localized Cr/Au layers (4) on the negative photoresin by positive photoresin treatment
• chemically etching the Al layer for chemically removing the microneedle.

3. Use of microneedles obtained by the method described in claim 2 for monitoring and stimulating neurons in neurological procedures.
